# EUROPEAN PATENT SPECIFICATION

(11) **EP 2 179 699 B1**
(45) Date of publication and mention of the grant of the patent: **06.01.2016**
(21) Application number: 09252473.5
(22) Date of filing: 23.10.2009
(51) Int. Cl.: A61B 17/34

(54) **Surgical access assembly**
Anordnung für chirurgischen Zugang
Ensemble d'accès chirurgical

(30) Priority: 23.10.2008 US 107749 P; 09.10.2009 US 576295
(43) Date of publication of application: 28.04.2010
(73) Proprietor: Covidien LP, Mansfield, MA 02048 (US)
(72) Inventor: Wenchell, Thomas, Durham, CT 06422 (US); Racenet, David C., Middletown, CT 06457 (US); Ceniccola, Anthony L., Hamden, CT 06514 (US); Racenet, Danyel J., Middletown, CT 06457 (US); Neave, Nadia Fern, New Haven, CT 06513 (US); Bettuchi, Michael J., Middletown, CT 06457 (US); Uznanski, Margaret, Great Neck, NY 11023 (US); Martin, Kimberly E., Boulder, CO 80301 (US); Leach, John, Woodbridge, CT 06525 (US); Haig, Fiona Middlemiss, Cambridge, CB24 9JG (GB); Collier, Nicholas John, Cambridge, CB25 0BB (GB); Fleming, Alistair, Cambridge, CB23 6ER (GB); O'Prey, Cormac, Bishop Stortford Hertfordshire, CM23 4HH (GB)
(74) Representative: Soames, Candida Jane

(56) References cited:
- US-A1- 2005 137 460
- US-A1- 2007 060 884
- US-B1- 6 312 377
- US-B1- 6 451 054

## Description

### BACKGROUND

### 1. Technical Field

The present disclosure relates generally to devices and techniques for performing minimally invasive surgical procedures. More particularly, the present disclosure relates to a device that is configured and dimensioned for positioning within an intercostal space to facilitate access to an internal surgical work site with one or more surgical instruments.

### 2. Background of the Related Art

In an effort to reduce trauma and recovery time, many surgical procedures are performed through small openings in the skin, such as an incision or a natural body orifice. For example, these procedures include laparoscopic procedures and thoracic procedures, such as those that are performed to investigate, diagnose, and treat diseases. Throughout the present disclosure, the term "minimally invasive" should be understood to encompass any and all such procedures.

Specific surgical instruments have been developed for use during minimally invasive surgical procedures, and typically include a shaft with an end effector, or operating portion, that is positioned at a distal end thereof. Dependent upon the requirements of the particular procedure, the end effector may include, for example, graspers, clip appliers, staplers, and the like.

During the minimally invasive procedure, the clinician often creates an opening through the patient's body wall using an obturator or trocar, and thereafter, positions an access assembly, such as a cannula assembly, within the opening. The access assembly typically includes an elongate access sleeve that is configured and dimensioned to receive one or more of the above-mentioned surgical instruments such that the end effector can be positioned within an internal work site adjacent the tissue that is the subject of the procedure.

Unlike laparoscopic surgery, which requires insufflation of the abdominal cavity to provide an operative region for the clinician, thoracic surgery does not require the introduction of insufflation gas into the thoracic cavity. Thus, the design of access assemblies intended for use during thoracic surgery can be simplified since the presence of a seal, or valve, is not essential.

In minimally invasive thoracic surgeries, the access assembly is generally inserted into a space located between adjacent ribs that is known as the intercostal space. After penetration of the patient's body wall is accomplished, the obturator can be removed from the access assembly, and one or more surgical instruments can be inserted into the internal work site therethrough.

In the interests of facilitating visualization, the introduction of certain surgical instruments, and/or the removal of tissue specimens during minimally invasive thoracic procedures, it may be desirable to spread the adjacent ribs defining the intercostal space.

Additionally, during these procedures, firm, reliable placement of the access assembly is also desirable in order to allow the access assembly to withstand forces that are applied during manipulation of the instrument(s) inserted therethrough. However, reducing patient trauma during the procedure, discomfort during recovery, and the overall recovery time remain issues of importance.

The pre-characterising features of claims 1 and 6 are known from US 6,451,054. It is further known from US 2005/013760 to provide a retractor ring with resilient material or an inflatable bladder.

### SUMMARY

The invention is defined in claim 1.

In one aspect of the present disclosure, a surgical access assembly is disclosed that is configured and dimensioned for positioning within an opening in tissue which provides access to an intercostal space defined between a patient's adjacent ribs in order to facilitate the passage of a surgical instrument into an internal work site. The body portion has an inner liner formed from a first material and defining an internal space that is configured and dimensioned to removably receive the surgical instrument, and an outer liner that is positioned about the inner liner. The outer liner is formed from a second material having a lower durometer than the first material such that the outer liner is resiliently deformable to facilitate conformity with the intercostal space to minimize the application of force to the patient's tissue upon insertion and removal of the access assembly and manipulation of the surgical instrument within the internal space. The body portion of the access assembly includes one pair of substantially planar sidewalls and one pair of arcuate sidewalls.

In one embodiment of the disclosed access assembly, the body portion may include a substantially rectangular cross-sectional configuration.

The surgical access assembly may include a body portion with a proximal portion that defines a first transverse dimension, and a distal portion that defines a second, smaller transverse dimension. The configuration and dimensions of the proximal portion facilitate engagement with the patient's tissue to prevent passage of the access assembly entirely into the internal work site.

In another aspect of the present disclosure, a surgical access assembly is disclosed that is configured and dimensioned to facilitate access to an internal work site through an intercostal space defined between a patient's adjacent ribs. The surgical access assembly includes a body portion having a proximal portion and a distal portion, and defines an internal space that is configured and dimensioned to receive a surgical instrument. The body portion incorporates an inner membrane and an outer membrane defining a cavity. The inner membrane and the outer membrane are formed from a biocompatible material that is substantially impermeable such that the cavity is capable of retaining a fluid therein.

The surgical access assembly is repositionable between deflated and inflated conditions upon communication of the fluid to and from the cavity such that the access assembly is resiliently deformable to thereby facilitate conformity with the intercostal space in order to minimize the application of force to the patient's tissue upon insertion and removal of the access assembly and manipulation of the surgical instrument within the internal space.

The distal portion includes substantially planar sidewalls and one pair of arcuate sidewalls.

The access assembly may include proximal and distal portions, wherein the proximal portion is configured and dimensioned to prevent the access assembly from passing entirely into the internal work site, e.g., the proximal portion may define a first transverse dimension, whereas the distal portion may define a second, smaller transverse dimension.

As with the surgical access assembly discussed in connection with the aforedescribed aspect of the present disclosure, in one embodiment, it is envisioned that the body portion may include a substantially rectangular cross-sectional configuration.

In another aspect of the present disclosure, a method of facilitating access to an internal work site beneath a patient's tissue is disclosed. The method includes the steps of (i) forming an opening in the patient's tissue; (ii) advancing an access assembly through the opening in a deflated condition such that the access assembly is positioned within an intercostal space defined between the patient's adjacent ribs; (iii) inflating the access assembly via communication of a fluid into a cavity defined between inner and outer membranes of the access assembly, whereby the access assembly conforms to the intercostal space to facilitate secured positioning thereof; (iv) inserting the surgical instrument into the internal work site through the access assembly; and (v) performing a surgical procedure utilizing the surgical instrument.

The step of inflating the access assembly may include causing a proximal portion thereof to define a first transverse dimension greater than a second transverse dimension defined by a distal portion thereof such that the proximal portion is configured and dimensioned for engagement with the patient's tissue to prevent passage of the access assembly entirely into the internal work site.

Upon inflation, the body portion of the surgical access assembly may be caused to realize a substantially rectangular cross-sectional configuration to facilitate conformity with the intercostal space. The body portion may be caused to define substantially planar sidewalls to maximize available surface area for contact with the patient's tissue, and/or at least one pair of arcuate sidewalls.

In still another aspect of the present disclosure, a method of facilitating access to an internal work site beneath a patient's tissue is disclosed. The method includes the steps of (i)

In still another aspect of the present disclosure, a method of facilitating access to an internal work site beneath a patient's tissue is disclosed. The method includes the steps of (i) forming an opening in the patient's tissue; (ii) advancing an access assembly through the opening into an intercostal space defined between the patient's adjacent ribs such that an outer liner of the access assembly is resiliently deformed to facilitate conformity with the intercostal space; (iii) inserting a surgical instrument into the internal work site through the access assembly; and (iv) performing a surgical procedure utilizing the surgical instrument.

Preferably, the step of inflating the access assembly includes causing a proximal portion thereof to define a first transverse dimension greater than a second transverse dimension defined by a distal portion thereof such that the proximal portion is configured and dimensioned for engagement with the patient's tissue to prevent passage of the access assembly entirely into the internal work site.

Preferably, the step of inflating the surgical access assembly may include causing a distal portion thereof to realize a substantially rectangular cross-sectional configuration to facilitate conformity with the intercostal space.

Preferably, the step of inflating the surgical access assembly may include causing the distal portion thereof to define substantially planar sidewalls to maximize available surface area for contact with the patient's tissue.

Preferably still, the step of inflating a surgical access assembly includes causing the distal portion thereof to define at least one pair of arcuate sidewalls.

According to a further aspect of the disclosure, there is provided a method of facilitating access to an internal work site beneath a patient's tissue comprising the steps of:
forming an opening in the patient's tissue;
advancing an access assembly through the opening into an intercostal space defined between the patient's adjacent ribs such that an outer liner of the access assembly is resiliently deformed to facilitate conformity with the intercostal space;
inserting a surgical instrument into the internal work site through the access assembly; and
performing a surgical procedure utilizing the surgical instrument.

The surgical access assembly may include a body portion with an inner liner positioned in contact with the outer liner, wherein the inner liner is formed from a first material, and the outer liner is formed from a second material having a lower durometer than the first material. Upon positioning within the opening, the inner liner may establish a passageway to the internal work site that extends longitudinally therethrough. It is envisioned that the proximal portion of the access assembly may be caused to abut the patient's tissue to prevent passage of the access assembly entirely into the internal work site.

### BRIEF DESCRIPTION OF THE DRAWINGS

Various exemplary embodiments of the present disclosure are described herein below with reference to the drawings, wherein:

FIG. 1 is a front view illustrating a patient's skeletal structure with one embodiment of the presently disclosed surgical access assembly positioned between the intercostal space defined between the patient's adjacent ribs in accordance with the present disclosure;

FIG. 2 is a top, perspective view of the access assembly of FIG. 1;

FIG. 3 is a side, perspective view of the access assembly of FIG. 1 positioned within the intercostal space;

FIG. 4 is a side, perspective view of the access assembly of FIG. 1 positioned within the intercostal space with a surgical instrument inserted therethrough;

FIG. 5 is a front, perspective view of an alternative embodiment of the access assembly of the present disclosure;

FIG. 6 is a side, perspective view illustrating another embodiment of the access assembly positioned within the intercostal space;

FIG. 7 is a side, perspective view of an yet another embodiment of the access assembly; and

FIG. 8 is a cross-sectional view illustrating another embodiment of the access assembly positioned between within the intercostal space.

### DETAILED DESCRIPTION OF THE EMBODIMENTS

Various embodiments of the presently disclosed access assembly, and methods of using the same, will now be described in detail with reference to the drawings wherein like references numerals identify similar or identical elements. In the drawings, and in the following description, the term "proximal" should be understood as referring to the end of the access assembly, or component thereof, that is closer to the clinician during proper use, while the term "distal" should be understood as referring to the end that is farther from the clinician, as is traditional and conventional in the art. Additionally, use of the term "tissue" herein below should be understood to encompass both the patient's ribs, and any surrounding tissues.

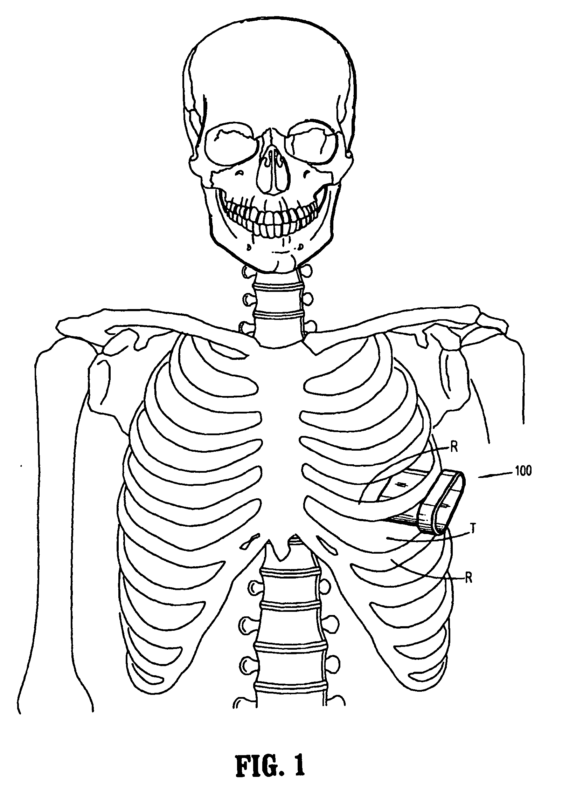

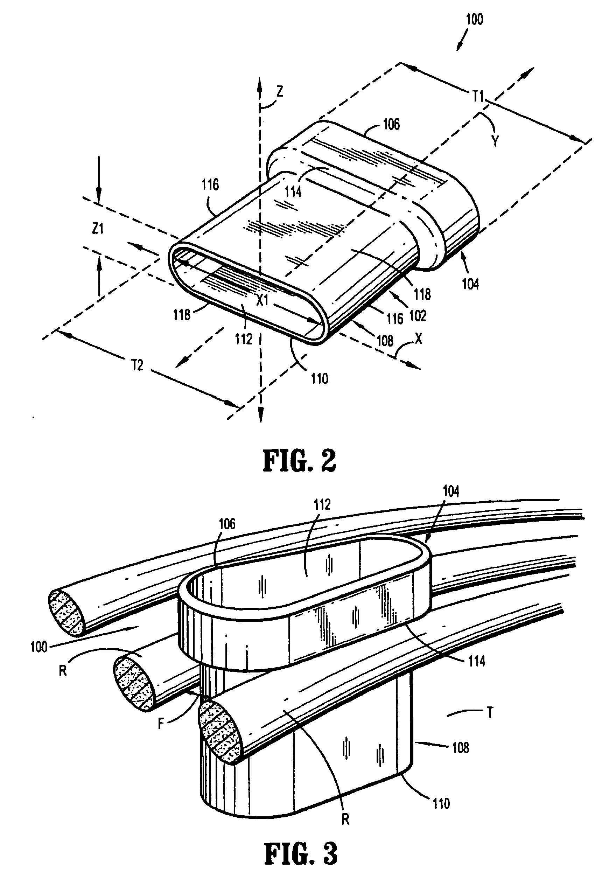

FIGS. 1-4 illustrate one embodiment of the presently disclosed surgical access assembly, which is identified by the reference character 100, in use during the course of a minimally invasive thoracic surgical procedure. As such, in the embodiment of the access assembly 100 seen in FIGS. 1-4, the access assembly 100 is depicted as a thoracic port that is configured and dimensioned for insertion into the intercostal space located between the adjacent ribs "R" of a patient in order to allow for the insertion and manipulation of one or more surgical instruments within the thoracic cavity "T" (FIGS. 1, 3). It is envisioned that the access assembly 100 may be formed from any suitable biocompatible material of a strength suitable for the purpose described herein, including, but not limited to, polymeric materials.

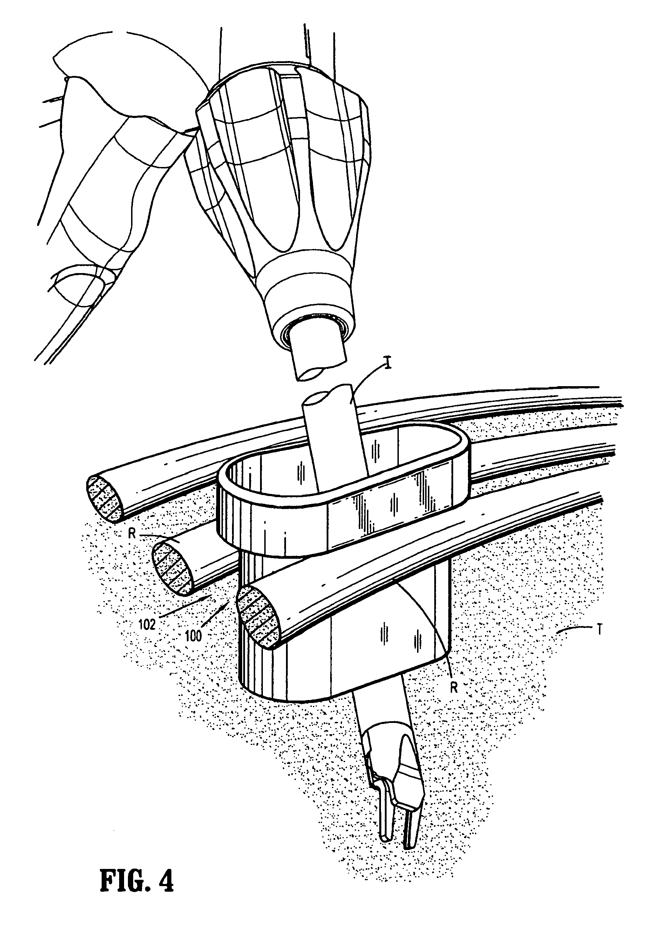

The access assembly 100 is configured and dimensioned to extend into the thoracic cavity "T" (FIGS. 1, 3) through the intercostal space, and includes a hollow body portion 102 (FIG. 2) that extends along a longitudinal axis "Y." The body portion 102 includes a proximal portion 104 with an open proximal end 106, a distal portion 108 with an open distal end 110, and defines an internal space 112 that is configured and dimensioned to receive one or more surgical instruments "I" (FIG. 4).

As best seen in FIG. 2, in one embodiment of the access assembly 100, the proximal portion 104 of the body portion 102 includes a transverse dimension "T1" that is larger than a transverse dimension "T2" defined by the distal portion 108. The larger transverse dimension "T1" of the proximal portion 104 facilitates manual engagement, e.g., gripping, by the clinician, and defines a flange, or buffer, 114 that is configured and dimensioned for abutment with the patient's tissue, e.g., the patient's ribs "R" (FIGS. 1, 3) during distal advancement of the access assembly 100 through the intercostal space. Contact between the flange 114 and the patient's tissue prevents the access assembly 100 from passing entirely into the thoracic cavity "T" (FIGS. 1, 3).

In one embodiment of the access assembly 100, the distal portion 108 of the body portion 102 includes a cross-sectional configuration defining a first transverse dimension "X1" that extends along a first transverse axis "X," and a second, smaller transverse dimension "Z1" that extends along a second transverse axis "Z," as best seen in FIG. 2. The disparity between the transverse dimensions "X1," "Z1" of the distal portion 108 allows the access assembly 100 to better conform to the configuration and dimensions of the intercostal space, while maximizing available space within the body portion 102 to facilitate the insertion and manipulation of the surgical instrument(s) "I" (FIG. 4), and minimizing the application of force "F" in the direction indicated in FIG. 3, e.g., to spread the patient's ribs "R." By minimizing the force "F," patient trauma is substantially reduced, as well as patient discomfort during recovery, and the overall recovery period.

The distal portion 108 of the body portion 102 includes a pair of first sidewalls 116 (FIG. 2), and pair of second sidewalls 118. In the embodiment of the access assembly 100 illustrated in FIGS. 1-4, the pair of first sidewalls 116 are illustrated as arcuate in configuration, whereas the pair of second sidewalls 118 are illustrated as substantially planar in configuration, whereby the distal portion 108 includes an elongated substantially, oval cross-sectional configuration. The substantially planar configuration of the pair of second sidewalls 118 maximizes the surface area available for contact with the patient's tissue.

In an alternative embodiment of the access assembly 100, it is envisioned that both the pair of first sidewalls 116 and the pair of second sidewalls 118 may be substantially planar in configuration such that the cross-sectional configuration of the body portion 102 is substantially rectangular.

The specific configuration and dimensions of the access assembly 100 ma y be varied in alternative embodiments of the present disclosure based on such factors as the anatomy of the patient to be treated, and the surgical instruments to be used in conjunction therewith. As such, it is further envisioned that both pairs of sidewalls 116, 118 may include arcuate profiles in order to further facilitate spreading of the patient's ribs, as well as maximization of the internal space 112 (FIGS. 2, 3) defined within the body portion 102.

With continued reference to FIGS. 1-4, use and operation of the access assembly 100 will be discussed during the course of a minimally invasive thoracic procedure. Initially, an opening is made in the patient's outer tissue wall of the thoracic body cavity by conventional means, such as by puncture via an obturator (not shown). Thereafter, the access assembly 100 is inserted through the opening into in the intercostal space between adjacent ribs, as shown in FIGS. 1 and 3. The access assembly 100 is advanced distally until the flange 114 defined by the proximal portion 104 is positioned in abutment with the patient's tissue, e.g., the patient's ribs "R," as shown in FIG. 4.

As mentioned above, it is envisioned that the body portion 102 (FIG. 2) may be dimensioned such that the patient's ribs "R" (FIGS. 1, 3) are spread apart, as indicated in FIG. 3, during distal advancement of the access assembly 100. If necessary, in order to further spread the patient's ribs "R," the access assembly 100 may be rotated, such as, for example, 90°. In order to maintain displacement of the patient's ribs "R" in the manner described, and/or the maintenance of suitable available space within the body portion 102 of the access assembly 100 to facilitate the insertion and manipulation of the surgical instrument(s) "I" (FIG. 4), it is envisioned that the material comprising the access assembly 100 may be of sufficient rigidity to resist excessive bending under the conditions normally encountered during such a surgical procedure.

Referring now to FIG. 4 in particular, the access assembly 100 is shown inserted into the thoracic cavity "T" (FIGS. 1, 3) between adjacent ribs "R" with a surgical instrument "I" inserted therethrough. The surgical instrument "I" may be any surgical instrument that is configured and dimensioned to pass through the body portion 102 of the access assembly 100, and adapted to perform a surgical, diagnostic, or other desired procedure. For example, suitable surgical instruments "I" may include endoscopic apparatus, which perform a variety of functions such as the application of surgical clips or other such fasteners to, and/or the cutting of, body tissue.

Following completed use of the surgical instrument "I," the instrument "I" can be withdrawn from the access assembly 100, and the access assembly 100 can be removed from the intercostal space.

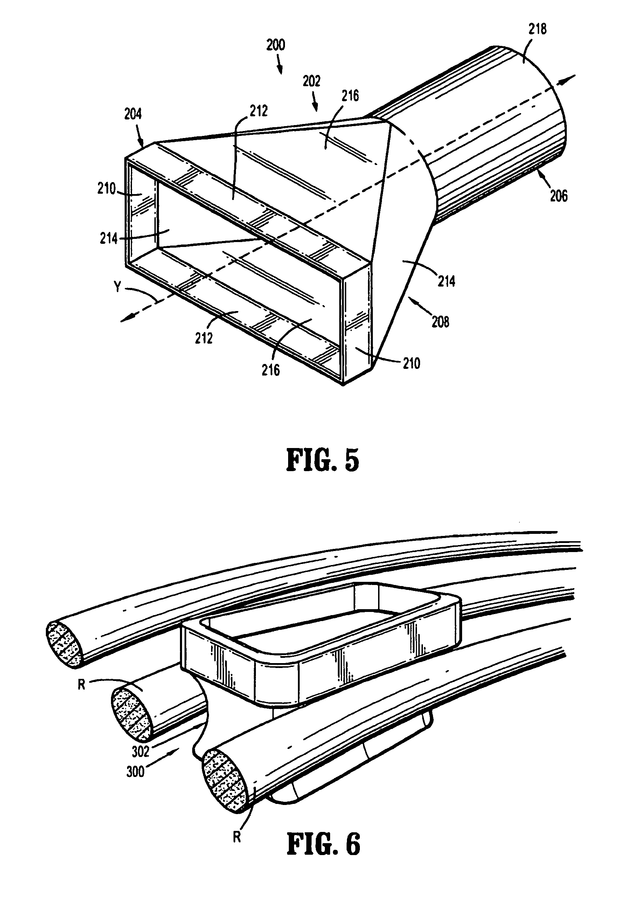

With reference now to FIGS. 5-8, alternative embodiments of the presently disclosed access assembly will be discussed. Each embodiment disclosed hereinbelow is substantially similar to the access assembly 100 discussed above with respect to FIGS. 1-4, and accordingly, will only be discussed with respect to any difference therefrom.

FIG. 5 illustrates an embodiment of the presently disclosed access assembly that is identified by the reference character 200. The access assembly 200 includes a body portion 202 having a proximal portion 204, a distal portion 206, and an intermediate portion 208 extending therebetween along a longitudinal axis "Y."

The proximal portion 204 includes respective first and second pairs of sidewalls 210, 212 that extend in substantially parallel relation such that the proximal portion 204 defines a substantially rectangular configuration. However, it should be appreciated that alternative configurations for the proximal portion 204, such as oval, are not beyond the scope of the present disclosure. The configuration and dimensions of the proximal portion 204 facilitates the insertion into, and the removal of the surgical instrument(s) "I" (FIG. 4) from, the access assembly 200.

The intermediate portion 208 extends distally from the proximal portion 204 and can be integral (monolithic) with the distal portion 206 and/or the proximal portion 204 or alternatively can be a separate element connected to the distal portion 206 and/or the proximal portion 204. The intermediate portion 208 includes respective first and second pairs of sidewalls 214, 216 corresponding to the sidewalls 210, 212 of the proximal portion 204. The first pair of sidewalls 214 taper in a distal direction inwardly towards the longitudinal axis "Y." The inward taper of the first pair of sidewalls 214 guides the surgical instrument(s) "I" upon insertion into the access assembly 200 to facilitate passage into the thoracic cavity "T", and provides a surface that is configured and dimensioned for abutment with the patient's tissue to prevent the surgical access assembly 200 from passing entirely into the internal work site, e.g. the thoracic cavity. Additionally, the tapered configuration of the first pair of sidewalls 214 provides increased internal space for manipulation of surgical instrument(s), e.g. instrument "I" of FIG. 4, and acts to distribute the load applied to the patient during insertion and manipulation of the surgical instrument(s) in order to reduce patient trauma during the procedure. The proximal portion has a larger transverse dimension to facilitate manipulation and to define a flange or buffer to prevent the access assembly from passing entirely into the thoracic cavity.

In the embodiment of the access assembly 200 illustrated in FIG. 5, the respective first and second pairs of sidewalls 214, 216 of the intermediate portion 208 are each illustrated as substantially planar in configuration. In alternative embodiments of the present disclosure, however, it is envisioned that the either or both of the respective first and second pairs of sidewalls 214, 216 may include an arcuate profile.

The distal portion 206 extends distally from the intermediate portion 208, and includes a wall 218 of a substantially cylindrical configuration defining an opening that extends therethrough. It should be appreciated, however, that the distal portion 206 may include other geometrical configurations, e.g., rectangular, in alternative embodiments of the present disclosure. The opening in wall 218 is configured and dimensioned to facilitate passage of surgical instrument(s), e.g. instrument "I" of FIG.4, through the patient's tissue and into the internal work site.

FIG. 6 illustrates another embodiment of the presently disclosed access assembly, which is identified by the reference character 300. In contrast to the aforedescribed embodiments, which are formed primarily from a substantially rigid material, the access assembly 300, and each variation thereof discussed hereinbelow, incorporates substantially compliant structure, either partially or wholly. The incorporation of compliant structure allows for reconfiguration of the access assembly 300 during insertion, removal, and manipulation of the surgical instrument(s), e.g. instrument "I" of FIG. 4. The ability of the access assembly 300 to be reconfigured during use maximizes the space available within the access assembly 300 for manipulation of the surgical instrument(s) positioned therethrough, and facilitates if desired more precise conformity with the shape of the intercostal space, thereby restricting movement of the access assembly 300 during the course of the surgical procedure, and consequently, any effect upon the patient's tissue that might otherwise result from such movement. Moreover, incorporating compliant structure into the access assembly 300 allows for a reduction in the forces applied to the patient's tissue during the course of the surgical procedure, if any, e.g., during insertion, removal, and/or manipulation of the surgical instrument(s), thereby reducing the influence of such forces upon the patient's tissues, and consequently, patient trauma, discomfort following the procedure, and recovery time.

The access assembly 300 includes a body portion 302 that is formed from the aforementioned compliant material, e.g., PU foam. The incorporation of such a material allows the body portion 302 of the access assembly 300 to better conform to the shape of the intercostal space defined between the patient's adjacent ribs "R." Additionally, the compliant material minimizes the force "F" (FIG. 3) applied to the patient's tissue during insertion and removal of the access assembly 300, which thereby minimizes any undesirable impact upon the patient's tissue. For example, the compliant material acts to dissipate any load transmitted to the intercostal nerve to further reduce patient trauma, discomfort following the procedure, and recovery time. The proximal portion has a larger transverse dimension to facilitate manipulation and to define a flange or buffer to prevent the access assembly from passing entirely into the thoracic cavity.

While illustrated as including a body portion 302 with a substantially rectangular in cross-sectional configuration, in alternative embodiments of the access assembly 300, it is envisioned that the body portion 302 may assume other geometrical configurations, e.g., oval. Also, the entire body portion 302 can be formed from compliant material, or alternatively, only portions thereof.

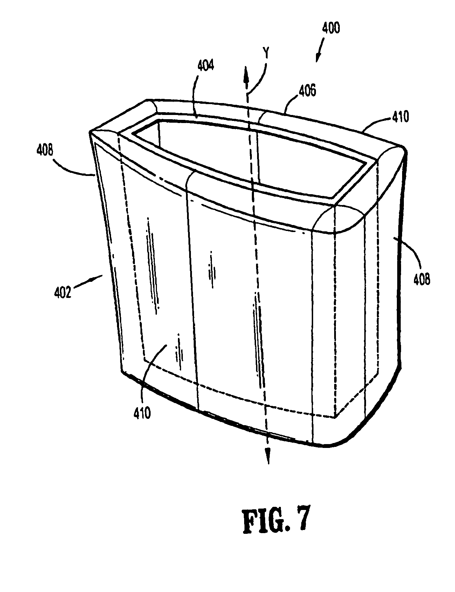

FIG. 7 illustrates another embodiment of the presently disclosed access assembly, which is identified by the reference character 400. The access assembly 400 includes a body portion 402 that extends along a longitudinal axis "Y" with an inner liner 404 and an outer liner 406 that is positioned thereabout. For example, it is envisioned that the outer liner 406 may be overmolded to the body portion 402, or that that inner liner 404 and the outer liner 406 may be connected through the use of an adhesive or other methods.

Whereas the inner liner 404 is formed from a substantially rigid material, the outer liner 406 is formed from a substantially compliant material, e.g., a material having a lower durometer than the material comprising the inner liner 404, either partially or wholly. By way of example, the outer liner can be formed from a biocompatible gel. Consequently, during use of the access assembly 400, the compliant outer liner 406 provides a cushioned contact area between the body portion 402 and the patient's tissue, e.g., the patient's ribs "R" (FIGS. 1, 3). Specifically, the patient's ribs "R," and the surrounding tissues, deform the outer liner 406 inwardly towards the longitudinal axis "Y."

Although the access assembly 400 is illustrated as including respective first and second pairs of first sidewalls 408, 410 that are substantially planar in configuration, whereby the body portion 402 includes a substantially rectangular cross-sectional configuration, alternative geometrical configurations for the access assembly 400 are not beyond the scope of the present disclosure. For example, it is envisioned that at least one of the respective first and second pairs of sidewalls 408, 410 may have an arcuate profile such that the cross-sectional configuration of the body portion 402 is substantially oval.

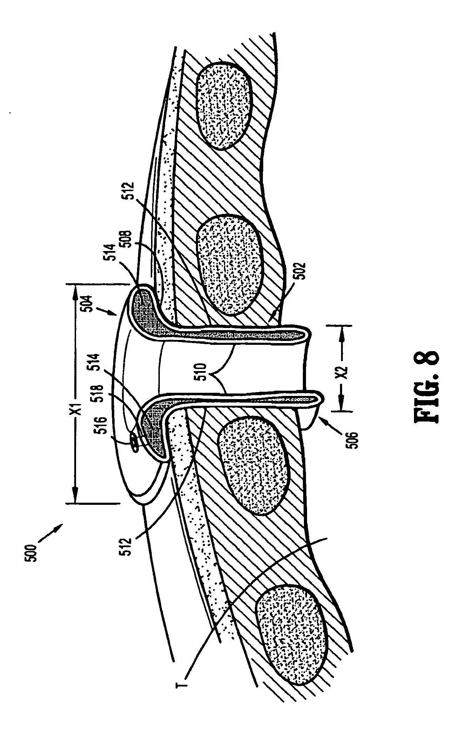

With reference now to FIG. 8, another embodiment of the presently disclosed access assembly, which is identified by the reference character 500, will be discussed. The access assembly 500 includes a body portion 502 having a proximal portion 504 and a distal portion 506. The proximal portion 504 defines a transverse dimension "X1" that is larger than the transverse dimension "X2" defined by the distal portion 506, whereby the proximal portion 504 defines a flange, or buffer, 508. The flange 508 is configured and dimensioned for engagement with the patient's tissue upon positioning of the access assembly 500 within the intercostal space, as shown in FIG. 8, in order to prevent the access assembly 500 from passing entirely into the thoracic cavity "T." In one embodiment, it is envisioned that the access assembly 500 may further include a distal flange (not shown) defining a transverse dimension larger than the transverse dimension "X2" defined by the distal portion 506 to facilitate engagement with a distal surface of the patient's tissue, e.g., in order to further enhance stability of the access assembly 500.

The body portion 502 includes an inner membrane 510 and an outer membrane 512 which collectively define a cavity 514. The respective inner and outer membranes 510, 512 may be formed from any suitable biocompatible material that is capable of retaining a fluid, e.g., air, water, or saline, within the cavity 514, i.e., a substantially impermeable material. During use of the access assembly 500, the cavity 514 is filled with the aforementioned fluid in order to transition the access assembly 500 between a deflated condition (not shown) and an inflated condition (FIG. 8). It is envisioned that the fluid may be introduced into the inner cavity 514 in any suitable manner, e.g., via a port 516 included on the outer membrane 512 that is in fluid communication with the inner cavity 514 via a lumen 518.

Inflating the access assembly 500 provides a measure of resiliency and deformability that cushions the patient's tissue during the course of the surgical procedure, and evenly distributes any applied forces, e.g., during insertion, removal, and/or manipulation of the surgical instrument(s), e.g. instrument "I" of FIG. 4. Additionally, the inflatability of the access assembly 500 allows the access assembly 500 to more precisely conform to the shape of the intercostal space. Conformity with the specific configuration and dimensions of the intercostal space minimizes the force "F" (FIG. 3) necessary to securely position the access assembly 500, and the need to separate the patient's ribs "R," which in turn, reduces patient trauma, as well as patient discomfort during recovery, and the overall recovery period.

Although the access assembly 500 is illustrated as including a substantially circular cross-sectional configuration, alternative geometrical configurations for the access assembly 500 are not beyond the scope of the present disclosure. For example, it is envisioned that the access assembly 500 may include substantially planar and/or arcuate sidewalls such that the cross-sectional configuration of the access assembly 500 is substantially rectangular.

Although described for use in thoracic procedures, it should also be understood that the access assemblies described herein can be used in other surgical procedures, including, but not limited to the replacement or reparation of a diseased or damaged valve, such as the repair and replacement of mitral, aortic, and other heart valves, repair of atrial and ventricular septal defects, pulmonary thrombectomy, treatment of aneurysms, electrophysiological mapping and ablation of the myocardium, and any other procedure in which interventional devices are introduced into the interior of the heart or a great vessel, as well as annuloplasty, quadrangular resection, commissurotomy, shortening mitral or tricuspid valve chordae tendonae, reattachment of severed mitral or tricuspid valve chordae tendonae or papillary muscle tissue, decalcification of valve and annulus tissue, and the like. The term "minimally invasive" as used herein also includes such procedures.

Persons skilled in the art will understand that the devices and methods specifically described herein and illustrated in the accompanying figures are non-limiting exemplary embodiments, and that the description, disclosure, and figures should be construed merely exemplary of particular embodiments. It is to be understood, therefore, that the present disclosure is not limited to the precise embodiments described, and that various other changes and modifications may be effected by one skilled in the art without departing from the scope of the disclosure. Additionally, it is envisioned that the elements and features illustrated or described in connection with one exemplary embodiment may be combined with the elements and features of another without departing from the scope of the present disclosure, and that such modifications and variations are also intended to be included within the scope of the present disclosure. Accordingly, the subject matter of the present disclosure is not to be limited by what has been particularly shown and described, except as indicated by the appended claims.

## Claims

1. A surgical access assembly configured and dimensioned for positioning within an opening in tissue providing access to an intercostal space defined between a patient's adjacent ribs to facilitate passage of a surgical instrument into an internal work site, the surgical access assembly comprising a body portion including one pair of substantially planar sidewalls and one pair arcuate sidewalls, **characterized in that** the body portion has an inner liner defining an internal space that is configured and dimensioned to removably receive the surgical instrument, and an outer liner positioned about the inner liner, the inner liner being formed from a first material, and the outer liner being formed from a second material having a lower durometer than the first material such that the outer liner is resiliently deformable to thereby facilitate conformity with the intercostal space to minimize the application of force to the patient's tissue upon insertion and removal of the access assembly and manipulation of the surgical instrument within the internal space.

2. The surgical access assembly of claim 1, wherein the outer liner is formed from a biocompatible gel.

3. The surgical access assembly of claim 1 or claim 2, wherein the outer liner is overmolded to the inner liner.

4. The surgical access assembly of any preceding claim wherein the body portion of the access assembly includes a substantially rectangular cross-sectional configuration.

5. The surgical access assembly of any one of the preceding claims, wherein the body portion includes a proximal portion defining a first transverse dimension, and a distal portion defining a second, smaller transverse dimension, the proximal portion being configured and dimensioned for engagement with the patient's tissue to prevent passage of the access assembly entirely into the internal work site.

6. A surgical access assembly configured and dimensioned to facilitate access to an internal work site through an intercostal space defined between a patient's adjacent ribs, the surgical access assembly comprising a body portion having a proximal portion and a distal portion, the body portion defining an internal space configured and dimensioned to receive a surgical instrument, and the distal portion including substantially planar sidewalls and one pair of arcuate sideweals, **characterized in that** the body portion comprises an inner membrane and an outer membrane defining a cavity therebetween configured and dimensioned to retain a fluid therein, the inner membrane and the outer membrane being formed from a substantially impermeable biocompatible material, wherein the surgical access assembly is repositionable between deflated and inflated conditions upon communication of the fluid to and from the cavity to facilitate resilient deformation of the access assembly and conformity with the intercostal space to minimize the application of force to the patient's tissue upon insertion and removal of the access assembly and manipulation of the surgical instrument within the internal space.

7. The surgical access assembly of any one of the preceding claims, wherein the proximal portion defines a first transverse dimension, and the distal portion defines a second, smaller transverse dimension such that the proximal portion is configured and dimensioned for engagement with the patient's tissue to prevent passage of the access assembly entirely through the intercostal space.

## Patentansprüche

1. Eine chirurgische Zugangsanordnung, die konfiguriert und ausgelegt ist zum Positionieren in einer Öffnung in einem Gewebe, die Zugang zu einem interkostalen Raum bereitstellt, der zwischen benachbarten Rippen eines Patienten definiert ist, zum Ermöglichen einer Durchführung von einem chirurgischen Instrument in eine innere Arbeitsstelle hinein, wobei die chirurgische Zugangsanordnung einen Körperteil aufweist, der ein Paar von im Wesentlichen ebenen Seitenwänden und ein Paar von gekrümmten Seitenwänden umfasst, **dadurch gekennzeichnet, dass** der Körperteil eine innere Auskleidung hat, die einen inneren Raum definiert, der konfiguriert und ausgelegt ist zum entfernbaren Empfangen des chirurgischen Instrumentes, und eine äußere Auskleidung hat, die um die innere Auskleidung herum positioniert ist, wobei die innere Auskleidung aus einem ersten Material gebildet ist und die äußere Auskleidung aus einem zweiten Material gebildet ist, das einen geringeren Durometer als das erste Material hat, so dass die äußere Auskleidung elastisch verformbar ist, um dadurch Übereinstimmung mit dem interkostalen Raum zu ermöglichen, um das Aufbringen von Kraft auf das Gewebe des Patienten beim Einfügen und Entfernen von der Zugangsanordnung und beim Betätigen von dem chirurgischen Instrument innerhalb des inneren Raumes zu minimieren.

2. Die chirurgische Zugangsanordnung von Anspruch 1, wobei die äußere Auskleidung aus einem biokompatiblen Gel gebildet ist.

3. Die chirurgische Zugangsanordnung von Anspruch 1 oder Anspruch 2, wobei die äußere Auskleidung eine Umspritzung der inneren Auskleidung ist.

4. Die chirurgische Zugangsanordnung von einem vorangegangenen Anspruch, wobei der Körperteil von der Zugangsanordnung eine im Wesentlichen rechteckige Querschnittskonfiguration umfasst.

5. Die chirurgische Zugangsanordnung von einem der vorangegangenen Ansprüche, wobei der Körperteil einen proximalen Teil umfasst, der eine erste transversale Abmessung definiert, und einen distalen Teil umfasst, der eine zweite kleinere transversale Abmessung definiert, wobei der proximale Teil konfiguriert und ausgelegt ist zum Eingriff mit dem Gewebe des Patienten zum Verhindern einer Durchführung von der Zugangsanordnung ganz in die innere Arbeitsstelle hinein.

6. Eine chirurgische Zugangsanordnung, die konfiguriert und ausgelegt ist zum Ermöglichen eines Zugangs zu einer inneren Arbeitsstelle durch einen interkostalen Raum, der zwischen benachbarten Rippen eines Patienten definiert ist, wobei die chirurgische Zugangsanordnung einen Körperteil aufweist, der einen proximalen Teil und einen distalen Teil hat, wobei der Körperteil einen inneren Raum definiert, der konfiguriert und ausgelegt ist zum Empfangen eines chirurgischen Instrumentes, und der distale Teil im Wesentlichen ebene Seitenwände und ein Paar von gekrümmten Seitenwänden umfasst, **dadurch gekennzeichnet, dass** der Körperteil eine innere Membran und eine äußere Membran aufweist, die einen Hohlraum dazwischen definiert, der konfiguriert und ausgelegt ist zum darin Aufbewahren eines Fluides, wobei die innere Membran und die äußere Membran aus einem im Wesentlichen impermeablen biokompatiblen Material gebildet sind, wobei die chirurgische Zugangsanordnung bei Übertragung von dem Fluid in und aus dem Hohlraum zwischen entleerten und aufgefüllten Zuständen repositionierbar ist, um eine elastische Verformung von der Zugangsanordnung und eine Übereinstimmung mit dem interkostalen Raum zu ermöglichen, um das Aufbringen von Kraft auf das Gewebe des Patienten beim Einfügen und Entfernen von der Zugangsanordnung und beim Betätigen von dem chirurgischen Instrument innerhalb des inneren Raumes zu minimieren.

7. Die chirurgische Zugangsanordnung von einem der vorangegangenen Ansprüche, wobei der proximale Teil eine erste transversale Abmessung definiert, und der distale Teil eine zweite kleinere transversale Abmessung definiert, so dass der proximale Teil konfiguriert und ausgelegt ist zum Eingriff mit dem Gewebe des Patienten zum Verhindern einer Durchführung von der Zugangsanordnung ganz durch den interkostalen Raum.

## Revendications

1. Ensemble d'accès chirurgical configuré et dimensionné pour être positionné dans une ouverture de tissu offrant un accès à un espace intercostal défini entre des côtes adjacentes d'un patient pour faciliter le passage d'un instrument chirurgical dans un site de travail interne, l'ensemble d'accès chirurgical comprenant une partie de corps comprenant une paire de parois latérales sensiblement planes et une paire de parois latérales arquées, **caractérisé en ce que** la partie de corps a une doublure interne définissant un espace interne qui est configuré et dimensionné pour recevoir de manière amovible l'instrument chirurgical, et une doublure externe positionnée autour de la doublure interne, la doublure interne étant formée d'un premier matériau et la doublure externe étant formée d'un second matériau ayant une dureté plus basse que celle du premier matériau de sorte que la doublure externe soit déformable de manière élastique afin de faciliter de la sorte la conformité avec l'espace intercostal pour minimiser l'application d'une force au tissu du patient lors de l'insertion et du retrait de l'ensemble d'accès ainsi que de la manipulation de l'instrument chirurgical dans l'espace interne.

2. Ensemble d'accès chirurgical selon la revendication 1, dans lequel la doublure externe est formée d'un gel biocompatible.

3. Ensemble d'accès chirurgical selon la revendication 1 ou la revendication 2, dans lequel la doublure externe est surmoulée sur la doublure interne.

4. Ensemble d'accès chirurgical selon l'une quelconque des revendications précédentes, dans lequel la partie de corps de l'ensemble d'accès comprend une configuration en coupe transversale sensiblement rectangulaire.

5. Ensemble d'accès chirurgical selon l'une quelconque des revendications précédentes, dans laquelle la partie de corps comprend une partie proximale définissant une première dimension transversale et une partie distale définissant une seconde dimension transversale plus petite, la partie proximale étant configurée et dimensionnée pour s'engager sur le tissu du patient afin d'empêcher le passage de l'ensemble d'accès intégralement dans le site de travail interne.

6. Ensemble d'accès chirurgical configuré et dimensionné pour faciliter l'accès à un site de travail interne à travers un espace intercostal défini entre des côtes adjacentes d'un patient, l'ensemble d'accès chirurgical comprenant une partie de corps ayant une partie proximale et une partie distale, la partie de corps définissant un espace interne configuré et dimensionné pour recevoir un instrument chirurgical et comprenant une paire de parois latérales sensiblement planes, **caractérisé en ce que** la partie de corps comprend une membrane interne et une membrane externe définissant entre elles une cavité configurée et dimensionnée pour y retenir un fluide, la membrane interne et la membrane externe étant formées d'un matériau biocompatible sensiblement imperméable, dans lequel l'ensemble d'accès chirurgical est repositionnable entre des états gonflé et dégonflé lors d'une communication du fluide avec la cavité ou en partant de celle-ci pour faciliter la déformation élastique de l'ensemble d'accès et la conformité avec l'espace intercostal afin de minimiser l'application d'une force au tissu du patient lors de l'insertion et du retrait de l'ensemble d'accès ainsi que de la manipulation de l'instrument chirurgical dans l'espace interne, la partie distale comprenant des parois latérales sensiblement planes et une paire de parois latérales arquées.

7. Ensemble d'accès chirurgical selon l'une quelconque des revendications précédentes, dans lequel la partie proximale définit une première dimension transversale et la partie distale définit une seconde dimension transversale plus petite de sorte que la partie proximale soit configurée et dimensionnée pour s'engager sur le tissu du patient afin d'empêcher le passage de l'ensemble d'accès intégralement à travers l'espace intercostal.
